# EUROPEAN PATENT APPLICATION

(11) **EP 2 179 751 A1**
(43) Date of publication of application: **28.04.2010**
(21) Application number: 08018399.9
(22) Date of filing: 21.10.2008
(51) Int. Cl.: A61L 27/56, C08F 2/44, C08J 9/00

(54) **Macroporous polymeric crosslinked materials, method for manufacture thereof and use**

(71) Applicant: Stichting Dutch Polymer Institute, 5612 AB Eindhoven (NL)
(72) Inventor: The designation of the inventor has not yet been filed
(74) Representative: Ackermann, Joachim

(57) **Abstract**

Disclosed is a method for generating macroporous polymeric crosslinked materials and the materials obtained therewith. The method uses beads of reversible hydrogels or selected core-shell beads as pore forming materials which are introduced as solid beads into a liquid of polymerisable monomers, for example via a drop-on-demand technique. After the polymerisation of the monomers the solid beads are liquefied and the pore-forming material is removed from the polymer matrix.

This technique allows formation of macropores with uniform diameter and with a high amount of macropores being in fluid communication with each other.

The macroporous polymeric crosslinked materials, preferably in the form of hydrogels, can be used as substrates in tissue-engineering.

## Description

This invention relates to an improved process for the manufacture of macroporous polymeric crosslinked materials, to macroporous polymeric crosslinked materials with improved properties and to the use of specific pore forming materials in the manufacture of macroporous polymerice crosslinked materials.

Macroporous crosslinked polymers are known in the art. These materials can be used in different fields of technology, for example as ion-exchangers, as filtration media, or as a substrate in biological applications, such as tissue engineering. A subgroup of macroporous crosslinked polymers includes hydrogels.

A "hydrogel" as two- or multicomponent systems consisting of a three-dimensional network of polymer chains and water that fills the space between macromolecules, which rheologically has a non-zero equilibrium modulus Gₑ. Such systems do not flow under the action of mechanical stress during the duration of experimental observations.

A hydrogel is said to be "reversible" when it reverts from a gel state to a sol state as a response to changes in the environment. Such an environmental change can be physical, such as a change in electric field strength, in magnetic field strength, applied pressure, in sound or light, or a change in chemistry of the surrounding environment, such as a change in pH, changes in ion concentrations as well as the addition enzymes, competing ligands, catalysts or other reagents.

Macroporous polymeric crosslinked materials, preferably hydrogels, show an unique combination of properties which makes them as potential candidates for several applications, such as agents for controlling drug delivery or as a substrate in tissue engineering.

All macroporous polymeric crosslinked materials - when combined with a liquid, such as an organic solvent or water - swell to a certain degree depending on the chemical composition of the polymer and the liquid. These swollen macroporous, polymeric crosslinked materials contain a continuous solvent phase and are continuously porous at the molecular / nanometer level. At least some of the pores in conventional macroporous polymeric crosslinked materials, especially in conventional hydrogels are closed cells. The size and uniformity of pores in these conventional materials are critical to applications like hydrogel-based scaffolds for tissue engineering, since they directy affect properties, such as mechanical strength and molecular permeability. With these conventional materials it is difficult to obtain networks of openly communicating pores.

One important property of macroporous polymeric crosslinked materials is the size and uniformity of pores as well as the communication between the pores in the polymer network. For tissue engineering a polymer, preferably a hydrogel is desired, wherein as much as possible of the pores formed within the polymer network are in fluid communication to each other thus forming an inner volume of the polymer. The number of isolated pores, of so called "closed cells" should be kept at a minimum and should be avoided, if possible. Materials with controlled, pre-programmed and preferably monomodal distribution of pore sizes would have beneficial properties, which would ensure, for example, maximum scaffold accessibility for cell migration or maximum control of release of active pharmaceutical ingredients.

Different approaches to the manufacture of macroporous polymeric crosslinked materials are known. Manufacturing methods comprise laser ablation, a layer-by-layer technique for the formation of polymers or the use of supercritical carbon dioxide as a pore-forming material, a so called porogen (see A. Cooper et al. in Advanced Materials, 11(15), 1270-4 (1999)).

Other approaches involve the addition of inorganic salts as porogens that are less soluble at a selected pH value and which are freely soluble at a different pH-value (see L. Gema et al. in J. Mat. Sci., Materials in Medicine, 15(4), 463-7 (2004)).
Yet another approach includes mixing a swater-soluble polymer porogen/diluent together with a pre-crosslinked hydrogel, allowing the hydrogel to become crosslinked and then removing the porogen/diluent to yield a more porous structure (see B. Manohar et al. in Biomaterials, 14(14), 1059-63 (1993)). Still another approach is the preparation of a hydrogel, swelling thereof, freezing of the swollen hydrogel and to allow the groving ice crystals to tear holes in the matrix (see K. Hye-Won et al. in Biomaterials, 20(14), 1339-44 (1999)).

WO-A-98/51,408 discloses hydrogel composites and superporous hydrogel compositions with fast swelling, high mechanical strength and superabsorbent properties. The hydrogels are manufactured using gas bubbles as a porogen and using disintegrant particles which are crosslinked to the polymer. The gas bubbles, for example carbon dioxide bubbles, are generated in situ and do not have a uniform distribution of diameters. Furthermore "superdisintegrants", such as Ac-Di-Sol^{R}, which is a crosslinked sodium carboxymethlycellulose, are added to maintain capillary channels in the sponge. But these materials are not removed after crosslinking of the polymer. During the crosslinking of the polymer the particles of the superdisintegrant swell, absorb monomers and yield a single, interpenetrating network around the bubbles of carbon dioxide.

These known techniques result in macroporous polymeric crosslinked materials with mostly closed cells and with a non-uniform distribution of pores sizes within the polymer network. For many applications, such as tissue engineering, it is highly desirable to have a polymer with a defined pore size and pore size distribution. In addition, it is highly desirable that as much pores as possible are in fluid communication with each other and form a defined an high proportion of inner volume that is readily accessible for reagent carrying fluids.

It is an objective of the present invention to provide macroporous polymeric crosslinked materials with a high amount of pores which are in fluid communication with each other.

Another objective of the present invention is the provision of macroporous polymeric crosslinked materials with well-defined and well-controlled pore size and with well defined and narrow pore size distribution.

Another objective of the present invention is the provision of a new and improved pore-forming material for the manufacture of macroporous polymeric crosslinked materials.

Another objective of the present invention is the provision of a simple and cost-efficient technique that allows formation of macroporous polymeric crosslinked materials.

The present invention relates to a process for producing a macroporous polymeric crosslinked material comprising the steps:
i) provision of a liquid containing at least one polymerisable monomer with polymerisable functional groups capable of forming a crosslinked polymer,
ii) addition of a plurality of separate beads to the liquid containing said at least one polymerisable monomer, said beads are made of a reversible hydrogel or said beads have a core-shell structure with a liquid core and a polymer shell, said polymer shell being insoluble in the surrounding liquid containing said at least one polymerisable monomer but being soluble in a suitable solvent,
iii) polymerisation of the at least one polymerisable monomer to form a crosslinked polymer in the presence of the beads as macropore forming materials,
iv) liquefaction of the beads, and
v) dissolution of the liquefied beads from the surrounding polymer matrix yielding macropores within the polymer matrix.

The term "polymeric crosslinked material" as used in this specification shall mean a polymer forming a three-dimensional network. The term polymer is used in its broadest sense. Polymers of this invention can be prepared by any polymerization reaction mechanism resulting in crosslinked polymers. The crosslinking can be performed by using monomers with two or more polymerizable groups in a molecule ("crosslinker monomer") optionally in combination with another crosslinker monomer or by using monomers with one polymerizable group in a molecule combined with at least one crosslinker monomer.

Hydrogels are known to the skilled artisan as homogeneously dispersed network of polymer chains that are swellable in an aqueous environment, where the polymer chains may be entangled, crosslinked, or ionically coordinated to each other to form a continuous netework. The term "hydrogel" as used in this specification shall mean a swellable colloidal dispersion of polymeric chains in water, which has a non-zero equilibrium modulus Gₑ.

The term "macroporous" as used in this specification shall mean the quality of a material containing networks of microchannels, formed by a series of pores that are in direct contact with one another, resulting in networks of pores that are in fluid communication with each other. The diameter of these microchannel forming pores is at least 1 µm, and preferably between 5 and 500 µm.

The term "addition of a plurality of separate beads to the liquid containing said at least one polymerisable monomer" as used in this specification shall mean any method that is capable to create a plurality of separate beads in the liquid of polymerisable monomer. The beads may be added to the liquid of polymerisable monomer or said beads may be formed within said liquid.

In a first embodiement the beads are made of a reversible hydrogel. In a second embodiement the beads have a core-shell structure with a liquid core and a polymer shell. The polymer used in the shell of the beads is insoluble in the surrounding liquid containing the at least one polymerisable monomer but is soluble in a suitable solvent for this polymer. In this embodiement the polymer can be a reversible hydrogel or it can be another polymer showing the above-defined solubility characteristics. In the method of this invention one or more different types of beads can be used.

One variant of preparation of a plurality of microdoplets of reversible hydrogel or of core-shell structure encompasses ejecting a solution of said hydrogel in a fluid sol state or of said polymer dissolved in the liquid forming the liquid core, into a liquid medium which rapidily switches the reversible hydrogel or the polymer forming the shell of the beads into a gel state. These micro-droplets may be ejected using several different inkjetting techniques, including both drop-on-demand techniques (e.g. thermo and piezoelectric-driven) as well as coutinuous jetting techniques (e.g. electrospraying and other methods based on Rayleigh jet breakup).

Preferably a plurality of microdrops of a solution of a reversible hydrogel in a solvent is added to the liquid of polymerisable monomers using a drop-on-demand technique to create a plurality of separate beads of reversible hydrogel in said liquid of at least one polymerisable monomer.

The liquid of polymerisable monomers in step i) can be any liquid which allows the formation of crosslinked polymers.

Preferably monomers are selected which allow the formation of crosslinked polymeric networks which are swellable in a selected solvent, usually, but not limited to, water. The prepolymerized monomer mixture should be compatible with the microgel beads, such that the microgel beads are stable before and during the crosslinking/polymerization of the monomer mixture, such that the microgel beads remain mostly in a gel state until crosslinking an/or polymerization reaction is complete.

As a liquid of polymerisable monomers mixtures of monomers can be used, which are liquid at the processing conditions of steps i), ii) and iii). As a preferred alternative a solution of polymerisable monomer mixtures in inert solvent or inert solvent mixtures are used, for example monomer mixtures in a hydrophilic solvent, such as water, alcohols, ethers or ketones or monomer mixtures in a hydrophobic organic solvent, such as hydrocarbons. Inert means here that there is no chemical reaction between the solvent, the monomers and formed copolymer during the polymerisation step. As another alternative oligomers with remaining polymerisable groups or polymers with remaining polymerisable groups can be used as polymerisable monomers, provided these oligomers or polymers are liquid at the processing temperature of steps i) and ii) or can form a solution in a suitable solvent.

In order to obtain crosslinked polymers which are swellable in a hydrophilic solvent the monomer or monomer mixtures to be polymerised should themselves be hydrophilic.

The monomers or monomer mixtures used in step i) can contain different chemical classes with polymerise with different chemical reactions. For example, monomers containing polymerizable olefinic bonds, polyamide-forming materials or an appropriate emulsion of polyoxetane or polyepoxide prepolymers may be used.

Preferably radically polymerisable monomers are used. These monomers in general contain at least two ethylenically unsaturated groups in the molecule.

All monomers used in step i) of the process of this invention can by hydrophobic (thus forming either no or very weak hydrogen bonds with water or phase separating from water and from related polar solvents); alternatively all monomers can be hydrophilic (thus being capable of forming thermodynamically favorable transient hydrogen bonds with water or being soluble or swellable in water and other polar solvents) or a combination of hydrophobic and hydrophilic monomers can be used. Furthermore, amphiphilic monomers can be used.

In a preferred variant of the process of this invention the polymerizable monomers are chosen to produce a macroporous crosslinked hydrogel.

In another preferred variant of the process of this invention at least one monomer must be hydrophilic, thus will form a water-swellable homopolymer when polymerised alone. This hydrophilic monomer can be combined with other copolymerisable monomers, for example with other hydrophilic monomers, or with hydrophobic monomers, which form non-water-swellable homopolymers when polymerised alone.

A preferred monomer is a difunctional α-unsaturated carboxylic acid ester, such as poly(ethylene glycol) dimethacrylate. Monomers with more polymerizable functional groups, such as trifunctional, tetrafunctional, pentafunctional or hexafunctional esters of α-unsaturated carboxylic acids, are also preferred, as well as polyfunctionalized macromonomers, such as methacrylate-functionalized polysaccharides.

Another preferred monomer is a difunctional unsaturated polysiloxane, for example a polysiloxane carrying terminal vinyl, allyl or ethylenically unsaturated carboxylic acid ester groups, such as poly(dimetylsiloxane) with terminal acrylate- or methacrylate groups. In this embodiement also monomers with three or more polymerizable functional groups can be present.

When polymerized alone, these multifunctional monomers form rigid, heavily crosslinked polymer networks. With the addition of some monofunctional comonomers, such ethylenically unsaturated acids, amides thereof or esters thereof the crosslinking density can be reduced, the swellability of the network in water increased, and the stiffness of the system becomes modified.

Examples of these monofunctional comonomers are acrylic acid, methacrylic acid, tiglic acid, vinylsulfonic acid, vinylphosphonic acid or the amides or the esters thereof, preferably the alkylesters thereof.

A preferred combination of monomers in step i) is a difunctional ester of an ethylenically unsaturated carboxylic acid and an at least trifunctional monomer which copolymerises with said difunctional ester. Alternatively the difunctional monomer can be added in step i) and the trifunctional monomer can be added after step ii) of the process. To this combination of monomers preferably one or more monofunctional monomers, for example an acrylate or a methacrylate or the corresponding carboxylic acids, can be added.

Very preferred in step i) a diacrylate of a polyethylene glycol and/ or a dimethacrylate of a polyethylene glycol is combined with a triacrylate of a trivalent alcohol, a trimethacrylate of a trivalent alcohol, a tetraacrylate of a tetravalent alcohol and/or a tetramethacrylate of a tetravalent alcohol.

Very preferred combinations of first and second polymerisable monomers are combinations of a diacrylate and/or a dimethacrylate of a polyalkylene oxide with a diacrylate and/or a dimethacrylate of a C₂-C₈ aliphatic alcohol without ether groups in the molecule.

In one embodiement of the process of this invention a plurality of separate beads of a reversible hydrogel is added to the liquid of polymerisable monomer(s). This can be advantageously performed by introducing a plurality of microdrops of a solution of a reversible hydrogel in a solvent into the liquid of polymerisable monomer(s). The microdrops can be formed by a drop-on-demand technique. After introduction of the microdrops into the liquid of polymerisable monomer(s) beads of reversible hydrogel are formed in said liquid thus the polymeric hydrogel forms polymer spheres of defined diameter or polymer particles of different shapes with defined size.

In another embodiement of the process of this invention a plurality of separate beads of core-shell structure is added to the liquid of polymerisable monomer(s). The shell of these beads is formed of a polymer what is insoluble in the liquid of polymerisable monomer(s) and the core of these beads is formed by a liquid which does not dissolve the polymer surrounding the core. The addition of the beads can be advantageously performed by introducing a plurality of microdrops of a solution of the shell forming polymer in a solvent into the liquid of polymerisable monomer(s). If this solution is printed into a non-solvent for the shell-forming polymer, for example into water, the shell-forming will precipate on the outer surface of the bead, forming a skin and the interior of the bead will stay liquid. Thus the exterior of the bead is a polymer film while the core is a liquid. As in the embodiment using reversible polymers as bead forming materials here also the microdrops can be formed by a drop-on-demand technique. After introduction of the microdrops into the liquid of polymerisable monomer(s) soft beads of core-shell structure having a polymer shell and a liquid core are formed in said liquid of polymerisable monomer(s) thus the core-shell beads form spheres of defined diameter or beads of different shapes with defined size.

In both embodiments, thus when using beads of reversible hydrogel or beads of core-shell structure, the size selection criteria for the beads is related to the intended application of the marcoporous polymeric crosslinked material, with the diameter of the spheres determining the lattice dimensions of the final scaffold. For cell culturing, for example, the scaffold must have an open lattice system with pores large enough for the cells of interest to migrate through, as well as provide space for the flow of nutrients or of oxygen into the system, and for the flow of metabolites and waste out of the system. The material must also supply mechanical support for the tissue.

The microdrops of the solution of reversible hydrogel or of shell-forming polymer can be introduced via the surface of the liquid of polymerisable monomer(s) or can be introduced directly into said liquid. The generation of microdrops of the solution of reversible hydrogel or of shell-forming polymer can be performed at a fixed location or the device forming the microdrops can be moved in a predetermined manner above the surface or within the volume of the liquid of polymerisable monomers.

In an alternative process the beads of reversible hydrogel or of core-shell structure having a polymer shell and a liquid core can be formed in a separate step and are subsequently introduced as beads into the liquid of polymerisable monomer(s), for example by adding a slurry of said beads in a non-solvent for the reversible hydrogel or for the shell forming polymer to said liquid of polymerisable monomer(s).

The formation of microdrops and their introduction into the liquid of polymerisable monomer(s) or into any other non-solvent for the reversible hydrogel or for the shell forming polymer can be performed by any drop-on-demand technique or by any other technique being capable of forming microdrops. Said drop-on-demand technique implies ejecting a discrete amount of liquid onto a specific location. An important feature of such drop-on-demand technique for the process of this invention is its ability to eject uniform droplets. Any jetting technique can be used that allows control of the droplet size. Examples of drop-on-demand techniques are drop-on-demand inkjet techniques, such as thermal, piezoelectric, electrostatic or acoustic inkjet techniques, or other techniques for creating microdroplets, such as pneumatic microvalve technology, double emulsion-solvent evaporation method or other emulsion methods. These technologies are known to those skilled in the art and are published, for example, in Tissue Engineering (2008), 14(1), 41-48, Pharm. Res. 8: 713-720 (1991) and Drug Development and Industrial Pharmacy (2001), 27(8), 825-829. Besides these methods continuous inkjet techniques can be used, for example binary deflection-, multiple deflection-, Hertz- and microdot-inkjet techniques. A preferred example of an applicable technique for step ii) is ink-jet printing, for example ink-jet printing using piezoelectric technique or using bubble-jet technique.

Inkjet printing enables microbeads to be prepared in general on the scale of 5 - 500 µm. If a macropore size smaller or lager than this is desired, a different drop formation technique may be required. Ink-jet printing is tpyically employed to eject spherical or semi-spherical droplets, but could also be used to prepare "strings" by adjusting the settings to release droplets at such a rate that they come into contact with one another while still in the sol state, coalescing into a single larger structure. Other microbead manufacturing techniques, such as pneumatice microvalve techology, double emulsion-solvent evaporation method or other emulsion method could be used either separately or in combination with ink-jet printing to create porogens of different dimensions and geometries.

Ink-jet printing is a preferred drop-on-demand method in the manufacturing process of this invention. This technique allows with high precision to rapidly dispense small aliquots of liquid. Size and shape of the droplets formed may be tuned by selecting the desired nozzle size, ink viscosity and droplet ejection conditions.

The beads of reversible hydrogel or of core-shell structure with polymer shell and liquid core can move by means of gravity to the bottom of the liquid of polymerisable monomer(s) or - depending on the viscosity of said liquid - may stay at the locus of formation. During introduction of the microdrops the liquid may be stirred to obtain an uniform distribution of the formed beads.

The amount of beads in the liquid of polymerisable monomers can vary within broad ranges and will be selected by the skilled artisan by the intended use of the final polymeric macroporous and crosslinked material.

Preferably the amount of beads chosen is so high enough that a major amount of the beads, for example more than 50 %, preferably more than 70 % and especially preferred more than 90 % are in direct contact with each other thus allowing the formation of macropores with fluid communication with each other. This means that at least a small portion of the surface of said macropores forms channels in said macropores thus creating a fluid communication of said macropores.

In a preferred embodiement of the process of this invention during of after step iv) the macroporous polymeric crosslinked material formed is subjected to mechanical shear-stress to cause opening and/or enlarging of channels between the macropores, for example by exerting an external mechanical stress to the body of the macroporous polymeric crosslinked material.

This process variation creates an additional amount of macropores that are in contact with other macropores which had been already in fluid communication with each other.

Theoretically, for uniformly-sized and efficiently packed spheres (i.e. face-centered), each sphere will be in contact with 12 surrounding spheres (i.e. will have a "kissing number" of 12). A packing density of approximately 0.7405 will be achievable for this scaffold. However, for random close packing, lower packing density are achieved, for example packing densities of approximately 0.64. These scaffolds, when compressed, will show higher average kissing numbers, for example average kissing numbers of 13.3. The packing density of the microspheres at the onset of polymerization is equal or almost equal to the empty volume of the final matrix.

It is also worth mentioning that one way of isotropically compressing the microspheres together (to improve surface contact and consequently microchannel communication) would be to swell them osmotically by switching the ionic concentration of the surrounding solution in a confined space (see, for example, European Physical Journal B: Condensed Matter Physics (1999), 9(1), 59-69). The spheres will absorb water from the surrounding solution until osmotic pressure is equilibrated. This will obviously increase the porogen packing density and kissing number.

It is also possible to used anisotropic compression to increase porogen packing density and kissing number. This could be performed, for example, by centrifugation or by loading the particles with a ferromagnetic particulate material, such as with particles of iron oxide, and exposing them to a magnetic field.

One advantage of the process of this invention is that beads with uniform diameter or a narrow distribution of diameters can be created.

Another advantage of the process of this invention is that the mean diameter of beads can be selected by proper adjustment of the microdrop formation conditions. The diameter of the microspheres determines the scaffold pore size, and is determined by the diameter of the ejected microdrops. In the case of piezoelectric inkjet printing, the size of the droples may be tuned by adjusting the printing settings, for example the nozzle diameter, the voltage applied to the piezo actuator, and the pulse width of the current.

The diameter of the microdrops can be selected, for example, in the range of 5 to 500 µm, preferably between 15 and 150 µm. The volumes of the droplets are preferably in the range of 10 pl to 14 nl per droplet.

The variation of the diameter of the individual microdrops in a given set of printing conditions is typically lower than 5 %, preferably lower than 2 %.

The high uniformity of microdrop diameter results in a high uniformity of the diameter of beads formed in the liquid of polymerisable monomer(s).

Besides one group of microdrops there can be different groups of microdrops with different mean diameters be introduced into the liquid of polymerisable monomers. This allows the formation of macroporous polymeric crosslinked materials with pores of defined different sizes.

Reversible hydrogels which can be used as pore-forming materials in the process of this invention are known to the skilled artisan. These polymers are capable of forming a solid hydrogel under defined conditions and can be liquefied under changed conditions. One non-limited group of reversible hydrogels are polymers wherin the sol-gel transition can be triggered by selecting temperature, pH-value, complexation or hydrogen bonding.

Examples of reversible hydrogels are swellable polymers with thermoreversible properties. Solutions of these polymers in suitable solvents form solids above a lower critical solution temperature ("LCST") as the polymer precipitates from the solvent. Below the LCST these polymers again form a solution in said solvent. Thus the gel-sol transition of these hydrogels is temperature dependent.

Further examples of reversible hydrogels are swellable polymers which can be crosslinked by using complexation and/or hydrogen bonding under defined conditions, for example by the addition of coordinations metal ions or at a defined pH-value. If solutions of these polymers in a suitable solvent are treated to form crosslinked materials the polymer precipitates from the solvent and forms a solid. If the pH-value is changed or if the coordinating metal ions are removed, for example by ion exchange, the polymers again forms a solution in said solvent. Thus the gel-sol transition of these hydrogels is dependent on pH-value or on the presence of absence of a complexing metal ion.

Still other examples of reversible hydrogels are swellable proteins which can form solid beads if introduced as a solution into a liquid that causes the solvent of the solution to become mixed with the liquid and said liquid causes the swollen protein to precipitate. If solutions of these proteins in a suitable solvent are introduced into the liquid of polymerisable monomer the proteins precipitate and form solid spheres. The precipitated protein can be re-liquefied by addition of enzymes which digest said protein into soluble portions of lower molecular weight and/or into amino acids. These products can be dissolved in a suitable solvent.

Additional other examples of reversible hydrogels involve the addition of protein-polysaccharide two-phase systems, whereby an thin layer of gel forms between e.g. calcium caseinate and sodium alginate, yielding a solid structure. These structures can be solubilized with the aid of digesting enzymes, such as trypsin, as well as calcium chelating agents such as EDTA. Calcium caseinate hydrogels, formed by treatment of casein with an acidic calcium solution, can be reversed by raising the pH.

Still other examples of reversible hydrogels are sodium caseinates that can be induced to gel at cold temperatures without the addition of divalent cations. Of course other physically crosslinked thermoreversible systems, such as collagen and gelatin can be used.

In a preferred process of this invention the reversible hydrogel is a hydrogel with thermo-reversible properties or a protein.

Preferred hydrogels with thermo-reversible properties are hydroxypropylcellulose, poly-(N-isopropylacrylamide), polyethyleneglycol-methacrylate, and/or poly-2-alkyloxazoline, polygalactose and/or agar which is/are added as aqueous solution or as alcohol solution to the liquid of polymerisable monomer(s).

Still other preferred hydrogels with ion-dependent properties are alginates. For example, if sodium alginate solutions are ink-jet printed into a bath containing calcium ions, the solution will undergo a metathesis process whereby sodium is replaced by calcium yielding an ionically-crosslinked hydrogel. Alginate is a linear copolymer composed of β-D-mannuronic acid and α-L-guluronic acid, and can be easily transformed into a gel by binding the guluronic acids with a divalent cation, such as calcium (see Biomedical microdevices 2007), 9(6) 855-862. The process can be reversed by the addition of a competing calcium chelating agent, such as EDTA.

Still other preferred hydrogels with pH-reversible properties are non-crosslinked polyacrylamides, polyacrylic acid and its salt and their block copolymers. This polymers are soluble at lower pH values and are insoluble at higher pH values and when reversibly crosslinked or entangled may form a network with non-zero equilibrium modulus.

Still other preferred reversible hydrogels are selected proteins which are gelatin and/or collagen and which is/are added as aqueous solution to the liquid of polymerisable monomers. These proteins are classic examples of materials capable of foming thermally reversible hydrogels, where polymer chains become entangled, but which can be reversed by heating.

An example of a system of core-shell forming soft beads is a solution of PLGA in an organic solvent for PLGA. If this solution is printed into water, the PLGA will precipate on the outer surface of the bead, forming a skin and the interior of the bead will stay liquid. Thus the exterior of the bead is a film of solid PLGA.

Once a sufficient amount of beads has been introduced into the liquid of polymerisable monomers, said monomers in said liquid are caused to polymerise and to form a crosslinked polymer network using the beads as a pore forming material. The polymerisation conditions depend on the nature of the polymerisable monomers, and can be selected by the skilled artisan using routine experiments.

For example, if radically polymerisable monomers containing ethylenically unsaturated groups are present, a radical forming material known as polymerisation initiator may be added or said initiator may be already be present and the conditions, such as the temperature of the liquid, may be altered in order to start the polymerisation.

Examples of radical initiators include, but are not limited to, organic peroxides such as dicumyl peroxide, 1,1-bis(tert-butylperoxy)cyclohexane, 2,5-di(tert-butylperoxy)-2,5-dimethyl-3-hexyne, benzoyl peroxide , 1,1-bis(tert-butylperoxy)cyclohexane, 1,1-bis(tert-butylperoxy)-3,3,5-trimethylcyclohexane, 2,2-bis(tert-butylperoxy)butane, 2,4-pentanedione peroxide, Luperox®, 2,5-bis(tert-butylperoxy)-2,5-dimethylhexane, 2-butanone peroxide, 3-chloroperbenzoic acid, benzoyl peroxide, bis[1-(tert-butylperoxy)-1-methylethyl]benzene, bis(tert-butylperoxyisopropyl)benzene, 1,1-bis(tert-butylperoxy)-3,3,5-trimethylcyclohexane, bis(tert-butylperoxyisopropyl)benzene, 2,5-bis(tert-butylperoxy)-2,5-dimethylhexane, bumene hydroperoxide, dicumyl peroxide, di-tert-amyl peroxide, ethyl 3,3-bis(tert-amylperoxy)butyrate, lauroyl peroxide, peracetic acid, tert-amyl peroxybenzoate, tert-amylperoxy 2-ethylhexyl, tert-butyl hydroperoxide, tert-butyl peracetate, tert-butyl peroxide, tert-butyl peroxybenzoate, tert-butylperoxy 2-ethylhexyl carbonate, or tert-butylperoxy isopropyl carbonate. inorganic peroxides, such as hydrogen peroxide, ammonium persulfate, hydroxymethanesulfinic acid monosodium salt dehydrate, potassium persulfate or sodium persulfate. These radical initiators can also be used in combination with a redox system, such as with iron-II-salts or with other redox agents. In addition to the peroxides mentioned, thermal initiators may be used, such as 1,1'-azobis(cyclohexanecarbonitrile), 2,2'-azobis(2-methylpropionamidine) dihydrochloride, 2,2'-azobis(2-methylpropionitrile), or 4,4'-azobis(4-cyanovaleric acid). In addition, organic photoinitiators, such as, 1-hydroxycyclohexyl phenyl ketone, 2,2-diethoxyacetophenone, 2-benzyl-2-(dimethylamino)-4'-morpholinobutyrophenone, 2-hydroxy-2-methylpropiophenone, 2-hydroxy-4'-(2-hydroxyethoxy)-2-methylpropiophenone, 2-methyl-4'-(methylthio)-2-morpholinopropiophenone, 3,6-bis(2-methyl-2-morpholinopropionyl)-9-octylcarbazole, 3'-hydroxyacetophenone, 4'-ethoxyacetophenone, 4'-hydroxyacetophenone, 4'-phenoxyacetophenone, 4'-tert-butyl-2',6'-dimethylacetophenone, acetophenone, 2,2-dimethoxy-2-phenylacetophenone, 4-(dimethylamino)benzoin, 4,4'-cimethylbenzil, benzoin ethyl ether, benzoin isobutyl ether, benzoin methyl ether, benzoin, [2-(acryloyloxy)ethyl]-trimethylammonium chloride, (4-benzoylbenzyl)trimethylammonium chloride, [2-(acryloyloxy)ethyl](4-benzoylbenzyl)dimethylammonium bromide, 2,5-dimethylbenzophenone, 2-methylbenzophenone, benzophenone-3,3',4,4'-tetracarboxylic dianhydride, 3,4-dimethylbenzophenone, 3-hydroxybenzophenone, 3-methylbenzophenone, 4-(diethylamino)benzophenone, 4-(dimethylamino)benzophenone, 4-(p-tolylthio)benzophenone, 4,4'-bis[2-(1-propenyl)phenoxy]benzophenone, 4,4'-bis(diethylamino)benzophenone, Michler's ketone, 4,4'-dihydroxybenzophenone, 5,5'-methylenebis(2-hydroxy-4-methoxybenzophenone), or benzophenone may be used. Moreover, thioxanthones such as [3-(3,4-dimethyl-9-oxo-9H-thioxanthen-2-yloxy)-2-hydroxypropyl]trimethylammonium chloride, 10-methylphenothiazine, 1-chloro-4-propoxy-9H-thioxanthen-9-one, 2,4-diethyl-9H-thioxanthen-9-one, or isopropyl-9H-thioxanthen-9-one may also be suitable.

After the polymerisation is finished the beads of reversible hydrogels or of shell-forming polymer are caused to liquefy. The measures for this depend on the specific type of reversible hydrogel or of shell-forming polymer used. In the case of a thermoreversible hydrogel, the solution may be liquefied by adjusting the temperature of the system to a regime to cause the reversible hydrogel to become liquefied in the solvent present in the polymerisation mass. This is preferably water. Other modes of re-liquefaction are explained above when describing the single types of reversible hydrogels. In the case of a thermo-reversible physical hydrogel, the solution may be liquefied by raising the temperature of the system above the gelation temperature.

The liquefied pore-forming material can be removed from the macroporous polymeric crosslinked material formed by the polymerisation reaction by rinsing with a solvent for said liquefied pore-forming material. As most of the macropores formed are in fluid communication with each other the solvent, preferably water, will flow through the pores and will remove the liquefied pore forming material.

In a preferred process of this invention a solution of reversible hydrogel with thermo-reversible properties is added to a liquid of polymerisable monomers, wherein during step ii) said solution and said liquid are a temperature above the lower critical solution temperature of the reversible hydrogel, the polymerisation of step iii) is performed at a temperature above the lower critical solution temperature of the reversible hydrogel and the liquidification of the beads of the reversible hydrogel is performed at a temperature below the lower critical solution temperature of the reversible hydrogel.

In another preferred process of this invention an aqueous solution of protein is added to a liquid of polymerisable monomers dissolved in a water-soluble organic solvent, the beads of protein are formed by dissolution of water into said solvent thereby causing said protein to solidify, the liquidification of the beads of the protein is performed by adding an enzyme that causes said protein to be digested into amino acids or into protein with smaller molecular weight, and the liquefied protein is dissolved from the polymeric crosslinked and macroporous material, preferably from a macroporous hydrogel-copolymer, by adding water to the crosslinked and macroporous polymer.

The process of this invention allows the creation of polymeric macroporous and crosslinked materials, preferably of macroporous hydrogels, with a high internal volume of macropores which are in fluid communication with each other. In prior art manufacturing techniques also polymeric macroporous and crosslinked materials including hydrogels can be produced. But these techniques allow only formation of materials with a limited portion of the macropores being in fluid communication to each other.

The macroporous system obtained by the process of this invention is chiefly different from known macroporous systems in terms of pore geometry. The close random packing of spheroids in the order of tens to hundreds of microns is a common model used by biologists for understanding cell aggregation (see for example Cytometry 28: 141-146 (1997)). But such system is difficult to achieve in a controlled manner using standard porogens, such as CO₂. The geometry of the products obtained according to this invention allows for greater microchannel communication and for greater pore content throughout the material, and mimics more closely the matrices found in living organisms.

In our experience macroporous polymeric crosslinked materials, preferably macroporous hydrogels, with a plurality of macropores within the bulk thereof, wherein at least 80 % of the macropores are in fluid communication with each other and wherein at least some of said macropores are in fluid communication with the surface of the polymer have not been disclosed until now.

The invention therefore also relates to these macroporous polymeric crosslinked materials, very preferably to such types wherein at least 90 % of the macropores are in fluid communication with each other.

The internal volume of the macropores may be determined by the use of optical microscopy, by electron microscopy or by nuclear magnetic resonance imaging.

Another object of the present invention is the use of a reversible hydrogel, preferable of a thermally reversible hydrogel, as a pore forming material in the manufacture of macroporous polymeric crosslinked materials, preferably in the manufacture of macroporous hydrogels.

Still another object of the present invention is the use of the macroporous polymeric crosslinked materials, preferably of macroporous hydrogels, defined above as a substrate for tissue engineering.

The following Examples illustrate the invention without any limitation.

### Example 1

Poly(vinyl alcohol-co-vinyl methacrylate) was prepared by dissolving 5 g. of polyvinyl alcohol (Fluka polyvinyl alcohol 8-88, MW 67,000; 0.114 moles repeating units) in dry THF (100 mL), and then adding triethylamine (1.17 g, 1.25 mL; 0.017 mol), followed by methacryloyl chloride (1.19 g, 1.27 mL; 0.011 moles). The solutions was stirred for 1 hr, and then filtered to remove triethylammonium chloride. The solvent was stripped off, and then the residue dissolved in 500 mL water and used as such as a 1 wt-% solution of poly(vinyl alcohol-co-vinyl methacrylate).

A 1% solution of poly-(lactide-co-glycolide) (PLGA; Sigma-Aldrich, 50:50 lactide:glycolide, Mw 40,000-75,000) in dichloroethane was prepared and printed using a 70 µm diameter glass micropipette, and printed into a 0.3% aqueous solution of polyvinyl alchohol (PVA 8/88 Fluka), using the following settings:
Printing settings: 65 V, 30 µs pw, 200 Hz freq.
Dispensing settings: 0.100 mm² x/y dot spacing, 100 dots² x/y

The dispensing pattern was repeated 100 times, yielding a mixture of the aqueous solution containing a precipitate of 100,000 beads. The beads were pipetted off the bottom along with sufficient solution around them to ensure that they were covered. A droplet of 1% lrgacure 369 in DMSO was added, and the mixture was cured using UV light. The material was soaked in THF, and the PLGA beads dissolved, yielding an open-pored structure which was analyzed using inverted light microscopy, and shown to have a nominal inner pore diameter of 25 µm.

### Example 2

A fresh batch of 1 % sodium alginate solution was made by slowly adding 1.0 g of sodium alginate to a beaker containing 100 ml of deionized water, and allowing it to dissolve for 30 minutes. The solution was filtered using a 1.2 µm-pore size nylon filter. The solution was printed into 1 ml of filtered 15 % CaCl₂ solution in a 15 ml centrifuge tube using a Microdrop ink jet printer equipped with an ADK glass micropipette with an internal nozzle diameter of 70 µm. The following settings were applied:
Printing settings: 150 V, 15 µs pw, 100 Hz frequ.
Dispensing settings: 0.200 mm² x/y dot spacing, 50 dots² x/y

The dispensing pattern was repeated 100 times, yielding a suspension containing 250,000 beads which precipitated to the bottom. The beads were transferred using a pipette to a second centrifuge tube, preloaded with 10 µL of a 50 wt.-% aqeous solution of polyethylene glycol dimethacrylate and 2 wt.-% Irgacure 369. This mixture was aspirated several times to encourage mixing, and the beads settled again to the bottom. The excess solution was removed from the top, and the remaining mixture was cured using UV light. The matrix was removed, and sliced using a microtone to expose the interior.

A solution of ethylenediaminetetraacetic acid (EDTA) was prepared by adding 10 g of ethylenediamine tetraacetic acid trisodium salt hydrate to 90 ml of distilled water, and mixing, yielding a nearly saturated solution. The matrix was exposed to the EDTA solution, and the beads dissolved within seconds, learving behind a matrix of interconnected pores which were measeurd using inverted light microscopy, and shown to have an average diameter of 42 µm.

## Claims

1. A process for producing a macroporous polymeric crosslinked material comprising the steps:
i) provision of a liquid containing at least one polymerisable monomer with polymerisable functional groups capable of forming a crosslinked polymer,
ii) addition of a plurality of separate beads to the liquid containing at lesat one polymerisable monomer, said beads are made of a reversible hydrogel or said beads have a core-shell structure with a liquid core and a polymer shell, said polymer shell being insoluble in the surrounding liquid containing said at least one polymerisable monomer but being soluble in a suitable solvent,
iii) polymerisation of the at least one polymerisable monomer to form a crosslinked polymer in the presence of the beads as macropore forming materials,
iv) liquefaction of the beads, and
v) dissolution of the liquefied beads from the surrounding polymer matrix yielding macropores within the polymer matrix.

2. The process as claimed in claim 1, wherein the macroporous polymeric crosslinked material is a macroporous hydrogel.

3. The process as claimed in claim 1, wherein the polymerisable liquid contains a monomer or a prepolymer with at least two polymerizable ethylenically unsaturated groups per molecule, which when polymerized yields a swellable crosslinked polymer network, preferably contains a difunctional ester of an ethylenically unsaturated carboxylic acid which forms a crosslinked and water-swellable homopolymer and an at least trifunctional crosslinker monomer and/or at least one monofunctional monomer which copolymerise with said difunctional ester.

4. The process as claimed in claim 3, wherein the difunctional ester of an ethylenically unsaturated monomer is a diacrylate of a polyethylene glycol and/ or a dimethacrylate of a polyethylene glycol and wherein the at least trifunctional monomer is a triacrylate, trimethacrylate, triacrylamide, trimethacrylamide of a of a trivalent alcohol or a trivalent amine, and/or a tetraacrylate or a tetramethacrylate of a tetravalent alcohol and/or a tetraacrylamide or a tetramethacrylamide of a tetravalent amine.

5. The process as claimed in claim 3, wherein the prepolymer with at least two polymerizable vinyl groups per molecule is a difunctional unsaturated polysiloxane, preferably a polysiloxane carrying terminal vinyl, allyl or ethylenically unsaturated carboxylic acid ester groups.

6. The process as claimed in claim 1, wherein the polymerisable liquid contains a first polymerisable monomer with two polymerisable groups forming a water-swellable homopolymer, a second polymerisable monomer with two polymerisable groups forming a non-water-swellable homopolymer and optinoally an additional monofunctional monomer, preferably wherein the first polymerisable monomer is a diacrylate and/or a dimethacrylate of a polyalkylene glycol and the second polymerisable monomer is a diacrylate and/or a dimethacrylate of a C₂-C₈ aliphatic alcohol without ether groups in the molecule, or is a diacrylamide and/or a dimethacrylamide of a C₂-C₈ aliphatic diamine without ether groups in the molecule.

7. The process as claimed in claim 1, wherein the reversible hydrogel is a hydrogel with thermo-reversible properties, with ion-reversible properties, or a protein, preferably a hydrogel with thermo-reversible properties selected from the group hydroxypropylcellulose, poly-(N-isopropylacrylamide), a polyethyleneglycol-methacrylate, a poly-2-alkyloxazoline a polygalactose and/or an agar which is/are added as aqueous solution or as alcohol solution to the liquid of polymerisable monomers.

8. The process as claimed in claim 7, wherein the solution of reversible hydrogel with thermo-reversible properties and the liquid of polymerisable monomers during step ii) are at a temperature above the lower critical solution temperature of the reversible hydrogel, the polymerisation of step iii) is performed at a temperature above the lower critical solution temperature of the reversible hydrogel and the liquefaction of the beads of the reversible hydrogel is performed at a temperature below the lower critical solution temperature of the reversible hydrogel.

9. The process as claimed in claim 7, wherein the reversible hydrogel is a hydrogel with ion-reversible properties which is an alginate, a polyuronate, preferably hyaluronic acid, a polyacrylamide, a polyacrylic acid, a sulfonated polystyrene or a sulfonated cellulose derivative.

10. The process as claimed in claim 7, wherein the protein is casein, fibrin, gelatin and/or collagen which is/are added to the liquid of polymerisable monomers in the form of an aqueous liquid substrate containing agents that cause the proteins gelate resulting in spheres with a water-insoluble surface.

11. The process as claimed in claim 10, wherein the aqueous solution of protein is added to a liquid of polymerisable monomers dissolved in a water-soluble organic solvent, wherein the beads of protein are formed by dissolution of water into said solvent thereby causing said protein to gelate, wherein the liquefaction of the beads of the protein is performed by adding an enzyme that causes said protein to be digested into amino acids or into protein with smaller molecular weight, and wherein the liquefied protein is dissolved by adding water to the macroporous polymeric crosslinked material.

12. The process as claimed in any one of the claims 1 to 11, wherein the plurality of separate beads of a reversible hydrogel is added to the liquid of polymerisable monomers in the form of a plurality of microdrops of a solution of a reversible hydrogel in a solvent using an addition technique to create a plurality of separate beads of reversible hydrogel in said liquid, preferably wherein the addition technique is a drop-on-demand ink-jet technique or a Rayleigh jet breakup technique.

13. The process as claimed in claim 1, wherein the amount of beads of reversible hydrogel in the liquid of polymerisable monomers is chosen to allow at least 80 % of all beads of reversible hydrogel to be in direct contact to each other, and preferably wherein during of after step iv) the macroporous polymeric crosslinked material formed is subjected to mechanical shear-stress to cause opening and/or enlarging of channels between the macropores.

14. A macroporous polymeric crosslinked material, preferably a macroporous hydrogel, with a plurality of macropores within the bulk thereof, wherein at least 80 % of the macropores are in fluid communication with each other and wherein at least some of said macropores are in fluid communication with the surface of said material.

15. A material of claim 14, wherein at least 90 % of the macropores are in fluid communication with each other.

16. Use of beads of reversible hydrogel and/or of beads of core-shell structure with a liquid core and a polymer shell, said polymer shell being insoluble in a liquid containing at least one polymerisable monomer, as a pore forming material in the manufacture of macroporous polymeric crosslinked materials, preferably of macroporous hydrogels.

17. Use of the macroporous polymeric crosslinked material of claim 16 as a substrate for tissue engineering.
